(19) Europäisches Patentamt
European Patent Office
Office européen des brevets

(11) **EP 4 491 125 A1**

(12) **EUROPEAN PATENT APPLICATION**

(43) Date of publication:
**15.01.2025 Bulletin 2025/03**

(21) Application number: **23184983.7**

(22) Date of filing: **12.07.2023**

(51) International Patent Classification (IPC):
**A61B 6/03** (2006.01)

(52) Cooperative Patent Classification (CPC):
**A61B 6/032; A61B 6/488; A61B 6/5288; A61B 6/545**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **NETSCH, Thomas**
**Eindhoven (NL)**

• **SCHMITT, Holger**
**Eindhoven (NL)**
• **FRERKING, Lena Christina**
**5656AG Eindhoven (NL)**
• **DESHPANDE, Hrishikesh Narayanrao**
**Eindhoven (NL)**
• **BERGTHOLDT, Martin**
**Eindhoven (NL)**
• **SENEGAS, Julien Thomas**
**Eindhoven (NL)**

(74) Representative: **Philips Intellectual Property & Standards**
**High Tech Campus 52**
**5656 AG Eindhoven (NL)**

(54) **SIMULATED 4D CT SURVIEWS USING 3D SURVIEW AND RGB-D CAMERA FOR IMPROVED SCAN PLANNING**

(57) System (FS) and related method for facilitating planning of raw data acquisition by a tomographic imaging apparatus (IA). The system (FS) comprises input interface (IN) through which is receivable input 3D surview image ($V0$) of a movable object (01) occupying a first portion of 3D space, the surview image ($V0$) previously obtained the tomographic imaging apparatus based on raw data acquired at a lower quality than a quality intended for the planned raw data acquisition, and the surview image representative of a first motion state of the object motion. A predictor component (PC) predicts, based on the input surview image($V0$), predicted 3D surview images ($Vj, j>0$) representable of the object in other, predicted, motion states, and the object in the said motion states occupying other portions of space. Output interface (OUT) provides the predicted surview images ($Vj$) for the said facilitating of the said planning of the raw data acquisition.

FIG. 3

EP 4 491 125 A1

**Description**

FIELD OF THE INVENTION

The invention relates to a system for facilitating planning of raw data acquisition by a tomographic imaging apparatus, to a related method, to an imaging arrangement, to a computer program element, and to a computer readable medium.

BACKGROUND OF THE INVENTION

CT (computed tomography) imaging typically begins with acquisition of surview images, also known as a scout or simply "surview". Such surview image is acquired prior to a diagnostic scan. Such surview may be used in assisting a user in adjusting a field-of-view ("FOV") of an imaging apparatus, such as the CT scanner. Such field of view may be understood as a subset in image domain, also called a "scan-box" for the given target anatomy.

As compared to a conventional 2D surview (coronal and sagittal views), today it is possible to acquire a 3D surview image, with similarly low X-ray dose as compared to the higher dose to be expended for the diagnostic scan. 3D surview imagery provides more detailed anatomical information as compared to 2D projection images. This information may be used for manual / automated planning of scans such as the selection of plan-box and safety margins.

Referring back to the 3D surview, these are static imagery, and do not provide temporal tissue motion caused by breathing or heart motion for example. Hence, planning of scan-box is only valid for a particular breathing state during the acquisition of the projection data. For example, when user plans a chest or heart anatomy, they may not know the true anatomy at some intermediate motion state in the breathing cycle. To compensate for this, a default safety margin is typically added to a tight scan-box, assuming that the organ of interest remains covered by such a larger scan-box also during changes induced by breathing. However, wrongly estimated margins may add unnecessary X-ray dose (too large a scan box), or unsatisfactory image quality with incomplete anatomical field-of-view (too small a scan box).

On another front, for workflow improvements in patient positioning, several vendors of tomographic modalities, such as CT or MRI (magnetic resonance imager/imaging), offer automated positioning based on RGB-D camera technology. The camera is usually mounted on a ceiling directly over the patient couch to monitor patient position and changes thereof during scan preparation. In addition to the ceiling mounted cameras used for positioning, in-gantry or in-bore cameras can be used to monitor patient during the raw data acquisition.

SUMMARY OF THE INVENTION

There may therefore be a need for improved planning in tomographic raw data acquisition.

An object of the present invention is achieved by the subject matter of the independent claims where further embodiments are incorporated in the dependent claims. It should be noted that the following described aspect of the invention equally applies to the related method, to the computer program element and to the computer readable medium.

According to a first aspect of the invention there is provided a system for facilitating planning of raw data acquisition by a tomographic imaging apparatus, comprising:

an input interface through which is receivable an input surview image ($V0$) of a movable object occupying a first portion of 3D (three dimensional) space, the surview image previously obtained by the, or a, tomographic imaging apparatus, based on raw data acquired at a lower quality than a quality intended for the planned raw data acquisition, and the surview image representative of a first motion state of the object motion;

a predictor component capable of predicting, based at least on the input surview image, one or more predicted 3D surview images ($Vj, j>0$) representable of the object in other one or more predicted motion states, and the object in the one or more predicted motion states occupying one or more other portions of space, and

an output interface capable of providing at least the one or more predicted surview images for the said facilitating of the said planned raw data acquisition.

The motion of which object of interest may be capable of is to be construed broadly. Thus, it may include any one or more of: translation, rotation, and deformation (eg, compression, contraction, expansion, etc). Such motion may include motion states. In each or some such states, the object my move to different space positions (relative to reference position), thus at least a part thereof occupying different portions of 3D space.

In a medical setting, the object of interest may be at least a part of whole of a target anatomical feature eg, at least a portion of a mammal (human or animal) heart, or at least a portion of at least one of the lungs, etc.

In embodiments, the system comprises a planner module capable of computing planning data based on the predicted one or more 3D surview images.

In embodiments, the planning data includes a specification of a target portion of the 3D space that includes the one or more other portions of space, and wherein the target portion of space is a subset of the input surview image.

The said target portion of space (in some imaging setting referred to as "scan box") includes/covers all voxel positions for which the projection data is to be acquired.

The target portion is preferably determined "tight", that is, it is the smallest portion of space that includes the (all of the) one or more other portions of space. Thus, the target portion is such that the moving object remains within the confines of the target portion during (all of) the motion.

In embodiments, the system comprises a graphics display generator capable for generating a graphics display for displaying on a display device, the generated graphics display capable of visualizing the one of more of the different motion states of the object based on the one or more predicted 3D surview images.

The display device may be integrated into operator console of imaging apparatus.

In embodiments, the graphics display includes video capable of representing an animation of the object transitioning between the different motion states, thereby occupying the different portions of space.

In embodiments, the system comprises a user interface capable to allow a user to designate the target portion in space based on the graphics display.

In embodiments, the predictor component is capable of computing the one or more predicted 3D surview images based on additional input data, including any one or more of: i) video footage acquirable of a surrogate feature in relation to the object by a non-ionizing radiation based camera, ii) a motion model for the said object motion. The camera may be optical, IR (infra-red), NIR (near IR), LIDAR (Light Detection and Ranging), laser, etc. The camera may be a depth sensing or ranging camera capable of recording over time varying depth profiles of the surrogate feature.

The surrogate feature may be an anatomic surrounding of the anatomical feature, such as a skin portion of chest, etc. In general, the object may be internal part of patient (the anatomical feature of interest), whilst the surrogate feature is external.

In embodiments, the motion model is a trained machine learning model trained on training data, such as artificial neural network, eg of the convolutional type.

The ML model may be of the generative type model and may allow predicting the "virtual" surviews without such camera channel. But non-generative ML such a regressor, eg of the supervised learning type, may be used. Thus, two main embodiments may be envisaged herein those of 1-channel input type (that use input surview) and the 2-channel input type that uses input surview and camera footage of associated motion of surrogate feature. ML model may be used with 1-channel or 2-channel embodiment.

For example, the training data may include instances (samples) of historical or synthetically generated imagery including: i) historical or synthetically generated 3D surview imagery of examples of the object and non-ionizing radiation-based surface footage of a respective example surrogate feature of the respective example object.

In addition, or instead, the training data may include sequences of such historical or synthetically generated surviews, from different patients that represent the object of interest in different motion states. The sequence can be split into sub-sequences, paired up one other surview sample to so obtain training pairs with one such surview as training input and the respective sub-sequence as target. Such pair may be use used to train regression model, in particular those configured for sequential data. Alternatively, or in addition, generative models may be trained based on such data.

In embodiments, the motion model represents at least one motion component of the motion as extractable from the video footage and wherein the predictor component to apply the so extracted motion component to the surview image to so extrapolate the predicted 3D surview images.

Rigid or elastic registration may be used to spatially register the input surview with the camera footage. This allows associating the two motions: motion of the object (target anatomical feature) as per the surview and the motion of the surrogate feature as per the camera footage.

In embodiments, the system may include a transducer capable of issuing an alert signal if the video footage of the non-ionizing radiation based (eg, depth sensing) camera is indicative of a deviation between the so imaged motion state and the model, and wherein the deviation is in violation of pre-defined acceptability condition.

In another aspect there is provided an imaging arrangement including the system of any one or more of the preceding claims, and any one or more of: the tomographic imager, the camera, the display device.

In yet another aspect there is provided a computing-implemented method of facilitating planning of raw data acquisition by a tomographic imaging apparatus, comprising ...

In yet another aspect there is provided a computing-implemented method of training, based on training data, a machine learning model for use in planning of a raw data acquisition by a tomographic imaging apparatus, the model, when trained capable of predicting, given a surview image of a movable object at a first motion state, one or more such predicted surview images for the object at different one or more motion states.

In yet another aspect there is provided a computing-implemented method of obtaining training data for use in a method of training, based on the training data to obtained, a machine learning model for use in planning of a raw data acquisition by a tomographic imaging apparatus, the model, when trained capable of predicting, given a surview image of a movable object at a first motion state, one or more such predicted surview images for the object at different one or more motion states.

The training data may be obtained by searching in a medical database, such as PACS, for patients with records that include surviews and at least one over time footage of surrogate feature. Alternatively, such surview vs surrogate feature video camera footage is generated in projects over one or more hospitals with cameras installed in-bore/in-gantry or such cameras being repurposed to obtain such pairs of surview vs camera footage.

In yet another aspect there is provided a computer program element, which, when being executed by at least one data processing system, is adapted to cause the data processing system to perform the any one of the methods.

In yet another aspect there is provided at least one computer readable medium having stored thereon the program element, or having stored thereon the model.

What is proposed herein in some embodiments is to combine information from the static 3D surview image with temporal motion information provided by a camera channel, such as a depth channel of a depth sensing camera, which may or may not include an optical RBG channel. Such cameras may be already fitted for patient positioning or surveillance during scan. It is proposed to use footage from such cameras for new purpose, namely scan box definition.

By using an explicit or implicit motion model (such as a breathing motion model), we propose to combine distance / range information from such, possibly already installed cameras, with information from the 3D surview image to generate an "animated" 4D surview, which is a prediction on how the original 3D surview image may change during the breathing cycle. The original static surview image is thus replaced by this animation, providing the user with additional valuable information for more accurate planning of the diagnostic scan.

In some aspects there is provided a system for facilitating planning of projection data acquisition by a tomographic imaging apparatus, comprising:

an input interface through which is receivable an input 3D surview image of an object occupying a first portion of 3D space, the surview image previously obtained based on projection data acquired at a lower dose than a higher dose for the planned projection data acquisition, and the surview image representative of a first motion state of the object capable of undergoing motion; and

a predictor component capable of predicting, based at least on the input surview image, planning data including a designated target portion in 3D space for which the imager is to acquire the projection data at the higher dose.

*"user"* relates to a person, such as medical personnel or other, operating the imaging apparatus or overseeing the imaging procedure. In other words, the user is in general not the patient. However, in some autonomous imaging setups, patient may perform some functions normally done by medical personnel or technician.

The terms *"object of interest"*, *"region of interest"* of *"target anatomical feature"* will be used herein interchangeably. This relates to the actual part of the human body or, in non-medical applications, that is the subject or purpose of the imaging, in particular of the diagnostic scan/high dose projection data acquisition. The feature which is imaged by the non-ionizing camera is not of diagnostic interest herein. Such feature (such a part of an anatomy, eg head, chest, torso, legs, etc) is a mere surrogate for the target anatomical feature, of main interest and purpose for the diagnostic scan. Such feature may be referred to herein as the, or a, surrogate feature, such as a surrounding (eg skin) of the anatomical feature or any other (external) anatomy imageable by non-ionizing camera. However, motion states of the surrogate feature may be correlated with motion states of the target anatomical feature.

*"motion state"* relates to the transitioning of object (such as TAF or SF) from position to position in 3D space. Motion may include any one, any two, or all three of translation, rotation, deformation. When an object, such as TAF or SF move, the object will occupy at least momentarily a different portion of 3D space than it did before. Such 3D space is defined by 3D coordinates (voxels). Thus, different voxels in image domain/examination region are so occupied in different motion states. An instant where a certain portion of space is so occupied is a "motion state". Thus, each motion state has a time and space component: the time at which object is occupying the respective portion of space, and the very space (subset in 3D) so occupied/filed. The space so occupied can be identified with the respective voxels, that is, with the set of spatial coordinates of the points in portion space so occupied.

*"scan box"* is a 3D subset of image domain. It comprises all voxels for which projection data/tomographic raw data is to be collected. The term "box" as used in "scan box" may indeed relate to a cuboid shaped such subset, this is not a requirement and "scan box" should be construed as any subset, whichever its shape. Preferably, the shape may be a function of the imaging geometry or of the type of coordinate system used in image domain. Scan box, or the voxel positions it comprises, may be defined in whichever form, as a subset of coordinates, as geometric loci of an equation, or in any form of parameterization, as is convenient and apt for the imaging task at hand.

*"raw data"* or tomographic raw data are measurement based on an interrogating signal interacting with patent tissue. Such signal may include X-ray in CT, RF signals in MRI, or gamma rays in PET. Such measurement are made at detector devices (X-ray or Gamma-ray sensitive detectors, or TRF coils) of tomographic imaging modalities such as CT, MRI or nuclear. Such detector devices define a data space, called projection or raw data domain, as opposed to another space, the image domain, in 3D made up of voxels (3D positions). Said voxels are populated by a tomographic algorithm (such as FBP or other) with tomographic image values that make up the reconstructed sectional image or volume in 3D. Projection data is one such example for raw data acquired in a tomographic setting, and this is in indeed mainly envisaged herein. In the following, reference is made mostly to such projection data, but the principles described herein in terms of planning data/scan box etc, are of equal application to other such tomographic imaging setups.

*"surview"* is general 3D and a reconstructed volume, reconstructed by use of a tomographic reconstruction algorithm, from raw data, such as projections. Image quality of such surview is lower than the (target) volume reconstructed from higher IQ raw data. Whilst such surview is of lower IQ than a diagnostic volume, some coarse details of anatomy (risk organs, abnormalities and prominent landmarks) are still discernable in, eg, low dose images. In the predicted virtual surviews, when run as animation, user may be able to discern coarse anatomy motion details, enough to plan the scan box.

In general, the term *"machine learning"* includes a computerized arrangement (or module) that implements a machine learning ("ML") algorithm. Some such ML algorithms operate to adjust a machine learning model that is configured to perform ("learn") a task. Other ML operate direct on training data, in which case the training data may form (at least part of) the model. This adjusting or updating of the model is called "training". In general task performance by the ML module may improve measurably, with training experience. Training experience may include suitable training data and exposure of the model to such data. Task performance may improve the better the data represents the task to be learned. Training experience helps improve performance if the training data well represents a distribution of examples over which the final system performance is measured. See for example, T. M. Mitchell, "Machine Learning", page 2, section 1.1, page 6 1.2.1, McGraw-Hill, 1997. The performance may be measured by objective tests based on output produced by the model in response to feeding the model with test data. The performance may be defined in terms of a certain error rate to be achieved for the given test data.

BRIEF DESCRIPTION OF THE DRAWINGS

Exemplary embodiments of the invention will now be described with reference to the following drawings, which, unless stated otherwise, are not to scale, wherein:

Fig. 1 shows an imaging arrangement including a tomographic imaging apparatus;
Fig. 2 is an illustration of the concept of a scan box as may be used herein as planning data;
Fig. 3 shows a schematic block diagram of a facilitator system for predicting planning data including such as a scan box;
Fig. 4 is an illustration of an embodiment of the facilitator system of Fig. 3 using explicit motion modelling;
Fig. 5 is a block diagram of a training system for training a machine learning model for use in implicit motion modelling in an embodiment of the facilitator system of Fig. 3;
Fig. 6 shows a flow chart of a computer/computing implemented method of facilitating definition of planning data of raw data acquisition by a tomographic imaging apparatus; and
Fig. 7 shows a flow chart of a computer/computing implemented method of training a machine learning model in implicit motion modelling for use in planning data definition.

DETAILED DESCRIPTION OF EMBODIMENTS

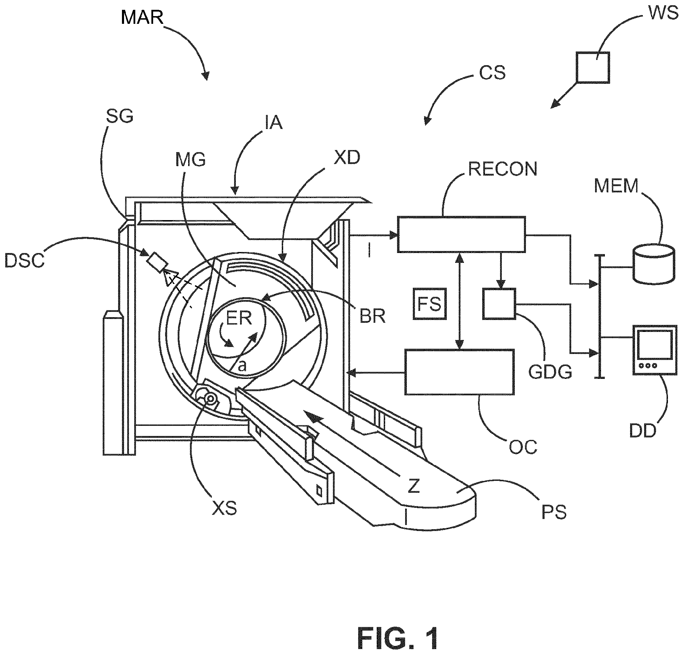

Reference is first made to Fig. 1 which shows a schematic block diagram of a medical imaging arrangement MAR as envisaged herein in embodiments. The arrangement MAR may include a medical imaging apparatus IA ("imager" for short), preferably of the tomographic X-ray based type, Thus the imager may be computed tomography (CT) scanner, but interventional systems such as those of the C-arm/U-arm-type are not excluded herein. Other tomographic modalities such as MRI, PET and others still are also not excluded herein in some other embodiments.

The arrangement IAR further includes a computing system CS which is broadly operable to process data, including image data, as provided by the imager. The computing system may further allow controlling operation of the imager. The computing system may be remotely located from the imaging apparatus IA, or may located close thereto, such as integrated into an operator console CS from which a user can operate the imaging apparatus, in particular can control an imaging operation to obtain medical imagery for diagnosis, treatment, planning (such as in radiation therapy, or other), etc.

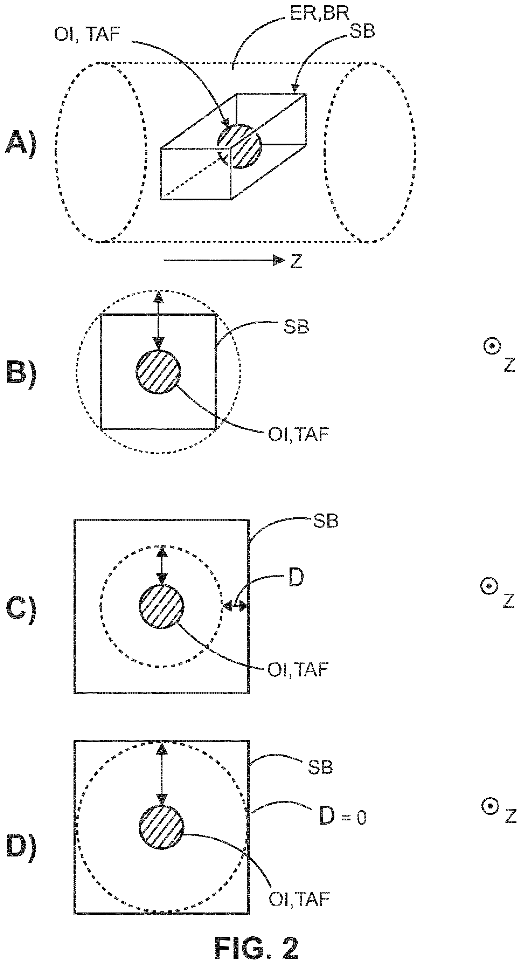

Broadly, and as will be explained more fully below, the computing system CS includes a facilitator system FS that may be used to facilitate planning data. The planning data may be used in a diagnostic scan to acquire sectional or volume data V of an object of interest OI, such as target anatomical feature TAF. The planning data ma included definition of a scan box SB, a concept explored below in more depth at Fig. 2. The scan box is data that allows controlling imager IA to acquire the right diagnostic tomographic raw data, such as projection data at a correct and appropriate dose for the imaging task at hand.

Broadly, the imaging may include two phases: a first planning (or preparatory phase), and a subsequent, second, operational phase. The facilitator system FS is mainly operable in the planning phase to obtain the scan box data. The scan box data may then be used in the operational phase to control imager IA to acquire, in the diagnostic scan, the diagnostic projection data $\lambda$ for the target image V.

The computing system CS, in particular its facilitator system FS, may be implemented as a Cloud solution, run on one or more servers. The imaging apparatus IA may be installed in a clinical facility, such as in a hospital for example. The computing system may be installed or used in a control room next to the imaging room where the imager IA is located. In some embodiments the computing system may be integrated into the imager IA. The imager IA may be communicatively coupled via a wired or wireless (or partly both) communication channel CC to computing system CS. The computing system CS may be arranged as a stationary computing system, such as a desktop computer, or as said server, or may be arranged as a mobile device, such as a laptop, smartphone, tablet, etc. For example, the computing system CS may be arranged on a work station WS associable with the imager IA.

Before explain operation of the facilitator system FS in more detail, reference is first mode to components of the imager IA to explain the two-phase imaging process in more detail, to so aid the later explanations in relation to the facilitator system FS.

The imaging apparatus IA is operable to generate, in particular acquire, projection data $\lambda$ which are forwarded through communication channel to the computing system CS for reconstruction into tomographic (sectional) images. The computing system CS runs one or more reconstructor units RECON, which implement one or plural reconstruction algorithms. In general, reconstruction algorithms implement a mapping that maps projection data $\lambda$ located in projection domain into image domain. Image domain is a portion of 3D space and is located in the examination region ER of the imaging apparatus, whilst the projection domain is in 2D, and is located at an (X-ray) detector XD of the imaging apparatus IA.

The image domain is conceptually made up of a 3D grid of 3D points (voxels), each with its own 3D coordinate $v(x,y,z)$ relative to a reference 3D coordinate system $(X,Y,Z)$ with an origin, which is assumed herein to be defined and given. Such reference coordinate-system with such origin is usually set up when the imager IA is installed in a calibration procedure. Some of the voxel positions v are populated by the reconstruction algorithm with values to so build up the surview volume image *V0,* or indeed any other scan such as the subsequent diagnostic target volume *V'*.

As said, the imaging apparatus IA is preferably of the tomographic type, and is preferably configured for multi-directional raw data acquisition , such as projection image acquisition. The imaging apparatus IA is thus capable of acquiring projection images $\lambda$ along different projection directions $\alpha$ relative to the examination region ER, and thus to the target anatomical feature TAF of/in patient PAT. Acquisition may be done in embodiments by a rotational system where at least the X-ray source XS is arranged in a moveable gantry MG.

The movable gantry MG (and with it the X-ray source XS in embodiments) is rotatable in a stationary gantry SG around the examination region ER in which the patient/ROI resides during imaging. Opposite the X-ray source in the movable gantry there is the X-ray detector XD which may rotate with the gantry and X-ray source around the examination region ER to realize the different projection directions $\alpha$.

As schematically indicated in Fig. 1, patient's longitudinal or imaging axis Z may extend into the examination region ER during imaging. Patient PAT may lie on a patient support PS, such as a bed, which is positioned at least partly in the examination region ER during imaging. In some, but not all embodiments, helical imaging protocols are envisaged herein where there is a relative lateral motion along the longitudinal axis Z between X-ray source XS and the patient PAT. For example, the patient support PS may be advanced through the examination region ER during the multi-directional projection image acquisition, for example during rotation of the X-ray source XS around the patient.

The CT scanner setup as illustrated in the Fig. 1 is merely according to one embodiment, and other tomographic imaging equipment, such as C-arm or U-arm scanners, Cone beam CT arrangements, mammographic imagers, etc, are not excluded herein. C-arm cone beam imagers are preferred herein in some embodiments. In addition, the multi-directional acquisition capability may not necessarily result from a rotational system such as shown in Fig. 1. Non-rotational imaging systems are also envisaged, such as in CT scanners of 4th or 5th generation, where multiple X-ray sources are arranged around the examination region in a source annulus for example. In addition or instead, the detector XD may be arranged as a detector annulus around the examination region. Thus, in such systems there is no rotation of the X-ray source XS or detector XD, or of both.

There may be an operator console OC through which a user, such as medical personnel, controls imaging operation. For example, user may request initiating the image acquisition or may request reconstruction or other operations, or may initiate transmission of data to the computing system CS, or may halt such transmission as required. The operator console OC may have integrated therein a display device for viewing control parameters, etc, but such in console display device may also be used herein by the facilitator system FS as will be explained later below.

During imaging, a respective X-ray beam XB issues forth, along the different projection directions $\alpha$, from a focal spot of the X-ray source(s) XS. The respective beam XB passes through the examination region with the patient in it. The x-radiation interacts with patient tissue. The X-ray beam XB is modified as a result of this interaction. In general, such X-ray beam XB modification includes attenuation and scattering of the original incoming X-ray beam. The modified X-radiation is then detected as a spatial distribution of various intensities at X-ray sensitive pixels of the detector XD.

It is not necessary herein to acquire projection images λ over a full 360° angular range around the examination region ER. Acquisition over a partial angular range such as a range of 270°, 180°, or even less may be sufficient. The X-ray detector is preferably configured for acquisition of 2D projection images with rows and columns of intensity values as registered by the detector pixels. That is, the detector pixels themselves may be arranged in a matrix layout. Such a 2D layout may be used in divergent imaging geometries, such as cone or fan beam or others. However, one dimensional detector pixel layouts (such as along a single line) are not excluded herein, and neither are parallel beam geometries.

The reconstructor RECON implements one or more reconstruction algorithms to process the diagnostic projection imagery λ. Specifically, the reconstructor RECON may compute tomographic target image V of the examination region (with the patient in it) for diagnostic, therapeutic or other purposes. The reconstructor RECON may be able to produce sectional volumetric image data ("image volume(s)") V. However, this does not exclude producing a single image slice in the examination region as required. Thus, the reconstructed imagery may be denoted herein as V which may include the whole volume, a partial volume or a specific section therethrough. Volumetric reconstruction may be facilitated by the helical movement and/or the 2D layout of the X-ray detector XD.

The scan box SB (data) may be defined automatically or by user in embodiment, in particular by using a user input UI functionality as supported by facilitator system FS. Broadly, the scan box CB is a portion of 3D space in §D image domain. It is thus a 3D object, but may be visualized in 2D view as a rectangular, hence the name scan box. The scan box defines the volume which is to be scanned in order to acquire projection data from which the volume of space demarked by the scan box is to be reconstructed.

The reconstructed volumetric imagery V may be stored in a memory MEM or may be otherwise processed, as required. The reconstructed volumetric imagery V may be visualized by a visualizer VIZ. The visualizer VIZ may generate a graphics display of the volume or a given slice. The graphics display may be displayed on a display device DD. The visualizer VIZ may map a section of image volume V, or the image volume V as a whole, onto a grey value or a color palette. The visualizer VIZ may control, via suitable interfaces, video circuitry to effect display of the graphics display on the display device DD. In addition or instead of so displaying, the reconstructed imagery V may be stored in a memory for later review or for other types of processing. Such a memory may include an image repository, such as a database (eg, PACS), or other (preferably) non-volatile data storage arrangement.

The reconstructed volume imagery V may be manipulated, for example by reformatting, to define cross-sectional planes that are different from the sectional plane defined by the imaging geometry. Such reformatting may allow medical user to better discern tissue type or anatomic details of the internals of the patient, in dependence on the medical purpose at hand, such as for diagnosis or preparation for some type of treatment, etc.

The so reconstructed target volume V is obtained from reconstruction of diagnostic projection data λ in the operational phase, based on information as per the scan box SB. This reconstructed image V is envisaged as a diagnostic image that represents the target anatomical feature TAF, such as a target anatomy, an organ, part of an organ, or group of organs, different tissue types, etc, and at sufficient contrast to safely inform therapeutic or diagnostic decisions, or other medical decisions such as to have further imaging sessions done, possibly using other imaging modalities, or other tasks (tests, etc) that need be done in light of the imagery.

The (target) projection imagery λ, from which the target volume V is reconstructed, is acquired at a sufficient dosage by suitably controlling via operator console OC the dose to be used. This may be done by controlling the voltage and/or amperage setting of the tube of the x-ray source XS to ensure a certain diagnostic image quality. Such target projection imagery acquired at sufficiently high dose, may also be referred to herein as the diagnostic projection imagery λ in order to follow established terminology. However, this naming convention is not to preclude that this projection data λ and its reconstruction V is to be used for tasks other than diagnostics, such as for therapy (in Cath Labs, etc), planning or any other task.

The target anatomical feature TAF relates to the medical purpose of the imaging. Thus, if patient's liver need examining, the target anatomical feature TAF is the liver: it is the liver that is the purpose and object of an abdominal scan to be done.

Each target anatomical feature TAF is in generally associated with an imaging protocol, a set of specifications, that stipulate, preferably as a function of patient bio-characteristics (age, weight, sex, height, BMI, medical record, etc), certain preferred imaging settings, the required image contrast to be achieved, the radiation dose to use for the target anatomical feature TAF for the given purpose, voltage/amperage settings of source X to use, collimation, etc. In short, imaging protocols encapsulate the medical knowledge for any given imaging task, purpose, target anatomical feature TAF, etc.

The facilitator system FS operates in the planning or preparatory phase, prior to the operation phase in which the target volume V is obtained.

In the planning phase, initial surview image V0 is obtained. This initial image V0 is preferably a 3D (image domain) surview volume, itself reconstructed by reconstructor RECON (or by a different reconstructor) from a first (non-diagnostic) set of projection imagery λ'. But this first /initial set of projection imagery λ0 is acquired in the planning phase in an first acquisition by imager IA at a lower image quality than the image quality of the projection imagery λ to be acquired later in the operational phase and to be used for reconstruction of target image V. Thus, there are two acquisitions: one in the planning phase to acquire low dose un-diagnostic projection data λ', and a subsequent acquisition in operational phase to acquire the higher dose diagnostic projection data λ.

In particular, when acquiring λ0, lower radiation dose is incurred as compared to the later dose that will fall due for the diagnostic scan to acquire the diagnostic projection imagery λ. This is to save dose on the patient PAT, and also because the 3D surview volume V0 serves an entirely different purpose than does the target volume V: the purpose of the surview volume/image V0 is essentially one of navigation, as will be expanded upon herein: the surview image V0 is used to define the scan box for the target anatomical feature TAF. The scan box thus controls imager to acquire the higher dose/higher dose diagnostic projection data λ for a smaller field of view (FOV) as per the scan box SB, focused on the target anatomical feature TAF. Thus, in the surview, a much larger FOV was used, but a lower dose. No planning is need there. However, once facilitator FS has defined the scan box, the diagnostic scan at a smaller FOV is run, but a higher dose to obtain the target volume for the target anatomical feature TAF at diagnostic grade higher IQ.

Referring now in more detail to the facilitator system FS, this is configured to facilitate definition of the scan box SB in respect of which diagnostic quality projection data λ is to be acquired in a new, prospective diagnostic acquisition by imager IA. Such diagnostic quality projection data λ is reconstructed by reconstructor RECON into the target volume V at an image quality ("IQ"), higher than the IQ of the low dose projection data λ0 based surview image V0. In operational phase, the new diagnostic acquisition by imager IA is expected to happen for the same patient, in the same exam room after the surview volume V0 has acquired, one the scan box has been computed by facilitator. This is the preferred workflow. Thus, the patient remains, preferably approximately in the same position, in the imager's bore BR for the surview scan and the later diagnostic scan. However, theoretically, if need be, it may be possible, for example with a suitable registration technique, to acquire the surview scan in first imaging session on one day, and have the patient come back for a later imaging session on another or the same day, and then acquire the diagnostic scan. However, such fragmented workflow is less preferred herein, and, as said the earlier option with patient PAT remaining in the imager's bore BR for both scans is preferred.

The target volume *V* confers a diagnostic quality view on the target anatomical feature TAF, or on any other object of interest OI. The object of interest OI will be referred to herein mainly as the target anatomical feature TAF, but both references will be used interchangeably herein. The term *"object of interest OI"* as used herein may be understood to refer also to foreign objects in the body (if any), such as implants etc, and may also be applicable in non-medical applications, also referred to herein in embodiments.

Whilst the following will be described with main reference to tomographic X-ray imaging, such as with an x-ray scanner of the doughnut type as shown in Fig. 1, or with C-arm scanners, or in any other design, the principles of scan box finding and definition as envisaged herein is not confined to x-ray based imaging, such as CT, but can also be practiced with benefit in other tomographic modalities, such as MRI, PET/SPECT, or any other where planning data need be established.

Before explaining operation of the facilitator FS, or "scan box definer", in more detail, reference is now made first to Fig. 2 which describe, in schematic fashion, the concept of scan box as may be contemplated herein.

Referring first to Fig. 2A, this shows a schematic view of the examination region ER, such as defined by imager IA's bore BR. As a CT imager is mainly envisaged herein, the examination region ER has the shape of a cylinder. The shape depends on the construction of the imaging apparatus, and hence may be other than cylindrical for other modalities. In MRI, the examination region ER is in general also of cylinder shape. The examination region ER may also be referred to as the imaging domain. As mentioned earlier, the image domain is a portion of 3D space, conceptually made up of the grid of 3D positions (voxels), as mentioned earlier.

The patient usually resides in the examination region, or it is at least the target anatomical feature TAF that resides in the examination region. The scan box is planning data that may be used for the diagnostic (eg, high dose) imaging of the target anatomical feature TAF. The scan box SB is a definition of a portion of 3D space. The scan box is 3D sub-portion of image domain. The scan box is a (mathematically true) sub-volume of examination region ER. Thus, the scan box SB is a (mathematically true) sub-set of the examination region. The scan box is so positioned and sized that it includes (covers) at least the target anatomical feature TAF. In yet other words, the scan box is a collection of voxel positions at which, at least at times, at least a part of the target anatomical feature TAF resides. The scan box can be defined by any geometrical shape. I does not necessarily have to be box (a cuboid) as shown in Fig. 2, but may be instead defined as an ellipsoid, a ball or may be of any other shape, depending on implementation. For example, the shape may depend in particular on the general image domain coordinate system (*X,Y,Z*). If the image domain coordinate system is Cartesian, as is often the case, a cuboid definition may be preferred for computational simplicity and low latency performance of the facilitator system FS. However, a ball shape definition or similar may be more appropriate if a polar coordinate system is used, etc.

The scan box SB defines a collection of voxel positions in respect of which projection data is to be collected in the coming up diagnostic projection data acquisition. The voxel positions form part of the 3D subset defined by the spatial confines of the scan box SB.

Once the scan box is sufficiently defined, it can be passed on to the control interface CL. The control interface CL translates the spatial information as per the scan box SB into imaging control commands or imaging parameters, in order to control machinery of the imaging apparatus IA to effect a corresponding projection data acquisition for all voxels within the scan box SB. Thus, the scan box defines such imaging control commands or imaging parameters. Such control operation may relate to the (relative) translation of the patient table PS and/or rotation of the X-ray source XS around the imaging axis Z. Such control operation may determine the location and length of a section (length) along the imaging axis Z, along and around which projection data is to be collected. The imaging control command or imaging parameters may also define other aspects of the imaging geometry, such as the beam shape to be used (eg, its collimation), start point of rotation of the X-ray tube, the pitch (a ratio of translation along axis Z and number of rotations around axis Z), and other imaging parameters or command, etc.

The scan box SB may be provided by the facilitator FS in any one of a number of different representations or specifications to the imaging control interface CL, all envisaged in different embodiments and depending on the control circuitry /code at hand. In general, and as will be apparent from Fig. 2A, the scan box is a specification of voxel positions. For example, the scan box SB may be supplied as a collection of all voxel positions that form the scan box. Alternatively, and preferably for low latency performance, the scan box may be supplied in a suitable parameterization. For example, coordinates of a reference point of the scan box may be provided, in addition to a size value. In more detail, for example, for a scan box in cuboid shape, a corner or center point may be supplied, long with a length of a diagonal of the cuboid. If ball shape is preferred, radius/diameter and center point may be supplied, etc. Alternatively, the reference point (such as corner point) and a length, width, and height of the scan box are so supplied. Any other parameterization, definition, of whichever kind, is envisaged, so long as it is unambiguously defined for each voxel position in the image domain/examination region. whether or not it forms part of the scam box.

The size of the scan box should be so that it includes the whole of the target anatomical feature TAF. However, if the target anatomical feature TAF is subject to motion such as one or more of the human lungs, parts of the heart, etc, care must be taken to define the size and position of the scan box accordingly to account for such motion. Thus, the anatomical feature TAF may be capable of motion such as translation, rotation, deformation and may assume different portions of space in the examination region in different motion states. The motion may or may not be periodic.

A tight definition of the scan box is preferred herein so that it includes all of the target feature TAF at all times during different motion states thereof, but should preferably be no larger than necessary as this may lead to unnecessary X-ray dose exposure of patient tissue irrelevant for the imaging task. Such tightness is hence a spatial minimization condition.

Figs. 2B, C illustrates what can go wrong when defining scan box SB of a target anatomical feature TAF subject to motion. The motion states are indicated by different line styles. The motion state indicated by the dashed line represents a different motion state as compared to motion state illustrated in solid line. Thus, the motion states represented in Figs 2B,C may be understood as an alteration of compression (Fig 2C) vs expansion (Fig 2B), such as of the human heart delivering its pumping function. As can also be seen in Figs 2B,C, when target anatomical feature TAF transitions in between different motion states, it occupies different portions of the surrounding space in image domain ER, thus occupying (or not) certain voxel positions. For clarity, the scan box SB in Figs 2B-D is illustrated along Z direction, that is, only along one spatial dimension.

The example scan box SB as illustrated by Fig. 2B is chosen too small. The target anatomical feature TAF in its motion, for example expansion, extends beyond the confines of the scan box. In the case illustrated in Fig. 2C on the other hand, the scan box is defined too liberally, leaving a clearance $\Delta$ of space which is undesirable, as dose is expended on voxel points in the clearance which do not provide any spatial information on the target anatomical feature TAF.

Fig. 2D illustrates the correctly chosen scan box that adheres to the minimization or tightness principle as mentioned above. Thus, boundary surfaces of the scan box SB are preferably chosen that they osculate boundary points on the target anatomical feature, in particular, those boundary points on the target anatomical feature TAF that travel the furthest as the target anatomical feature TAF transitions through its motion states. The "dead space" $\Delta$ of voxels that are never visited by the target anatomical feature TAF during its motion is preferably approximately zero. Thus, the are no or very few such points within scan box SB that are never so visited.

Thus, the facilitator FS is configured to facilitate spatially defining the scan box to ensure that all points of the target anatomical feature remain within the confines of the so defined scan SB box at all times, without there being voxels which are never visited by target anatomical feature TAF during its transitioning through different motion states. Thus, whilst the scan box itself remains static, it is defined in size and position so that the target anatomical feature TAF remains within the box at all times, with "dead space" $\Delta$ approximately zero as illustrated in the tightly defined example scan box SB of Fig. 2D.

Broadly, the facilitator system FS may assist the user in defining the voxel positions of the scan box. This can be done by defining the size and position of bounding box in image domain ER. The facilitator achieves this by processing as input the surview image *V0* acquired of the target anatomical feature TAF at a given (*"initial"* or *"reference"*) motion state, that is, at an initial position and extent of the target anatomical feature TAF. Thus, the target anatomical feature TAF at this initial motion state occupies an initial portion of 3D space in the examination region ER. The mentioned furthest motion state of the target anatomical feature TAF is taken relative to this initial motion state as recorded in the (initial) surview image *V0*. The surview image *V0* is volume data so captures the examination region ER in 3D at a suitably large extent, either over the whole examination region ER (whole body scan), or pat thereof that is a priori known to include the target anatomical feature TAF but in addition to other surrounding anatomies with substantial (at any rate non-zero) dead space ∆, thus proving surview anatomical information on how the target anatomical feature TAF is embedded in the surrounding overall/larger anatomy of patient.

The facilitator systems FS processes this surview image V0 as input data and produces a prediction of multiple "virtual" surview images Vj, based on the surview image V0. Preferably, each virtual surview Vj represents the target anatomical feature TAF at a different, predicated, motion state, different from the initial motion state as per V0. In the following, for the sake of brevity, we will refer to the realinitial and virtual surview images simply as "surview(s)", dropping *"volume"* or *"image"*. The virtual one or more surviews Vj computed by facilitator FS are preferably of the same size and dimension as the initial surview. Thus, the virtual surviews Vj are also volumes representative of 3D space within the examination region, capturing an image portion with dead space *⊿>0*. However, whilst the initial surview V0 is based on measured projection data, the virtual surview are predictions and are hence not, or at least not directly, based on such measured projection data. Thus, no further dose is incurred when producing the virtual surviews *Vj, j>0*. The predicted surview images *Vj* may be conceptualized as extrapolations from the initial input surview volume *V0*.

Preferably, the number of virtual surviews Vj so predicted for different time instants (represented by index *j*) are such that they cover all motion states of the objects, even those that reach furthest away from a pre-defined reference motion state represented/captured in the actual reconstructed input surview V0. Theoretically, and indeed envisaged in some embodiments, it may be sufficient to predict only one such virtual surview image *Vj\**. If such a single surview is predicted, this is preferably such that is represents the motion state (extremal motion state) of target anatomical feature that is spatially the furthest away from the initial motion state of the target anatomical feature as captured in the initially measured surview *V0* represents. Preferably, however, and in some embodiments more than one such predicted surview images are computed by the facilitator system to represent intermediate motions states inbetween the initial state as per initial surview V0 and the extremal motion state as predicted and represented in extremal surview *Vj\*,* as it may be aptly called herein. The plural such surviews Vj may be replayed in sequence as media footage (as an "animation") that can be displayed on a display device DD. Thus, the animation may be understood as conveying a visual sense of the target anatomical feature "wobbling", enough for the user to estimate from mere observation of the animation, a sufficiently large, yet tight, scan box. The animation may be rerun, in on or more replays, as often as needed, in a replay loop. Thus, the media footage Vj displayed as video/animation illustrates to user how the target anatomical feature TAF is expected to move as it transitions through the different motion states thereby occupying different spatial portions in the examination region. The display device may be integrated into the operator consoles OC of the imager IA.

The facilitator system FS may be capable of computing the scan box based on such output predicted surviews Vj, without any user involvement, in particular without displaying any of the so computed predicted or virtual surviews Vj in a medialvideo footage as mentioned above. Preferably, however, there is some user involvement by using a user interface UI. In such, more user centric, embodiments, one or more of the predicted surviews Vj are displayed either in conjunction with original V0 or instead thereof, and the user can then use a graphics tool as may be supported by the user interface UI to allow user essentially draw/outline, etc the scan box appropriately using the spatial information as per the displayed predicted surviews Vj. In particular, the mentioned media footage/animation can be rendered for view display device DD to provide the user with an illustration of the portion of space that are occupied by the target anatomical feature during its motion state transitions.

The user, after one or more repetitions of the video footage, may stop the animation at the point where the furthest position is reached in the associated extremal motion state, and then draw the scan box accordingly. The user interface UI may be implemented by touch screen or by pointer tool (mouse, stylus, etc) interaction. The user may draw a geometrical shape such as a rectangle in 2D or a cuboid in 3D to circumscribe, in a tight manner, the extremal motion state of the target anatomical feature TAF, with boundary surfaces or lines of the scan box SB as drawn tangential to the spatially most extremal point(s) of the target anatomical feature TAF as represented in the predicted extremal surview Vj*. The co-ordinates so specified by this drawing action are captured and stored as the scan box specification which may then be forwarded automatically or upon use request the control interface CL. The diagnostic scan based on the so specified scan box may then commence. Again, commencement of this scan my be automatic upon user dispatching the specified scan box, or scan is initiated up a user issued go-ahead signal. Because of the 3D nature as envisaged herein, the video footage replace of the predicted surviews Vj may be done along each spatial dimension X,Y,Z and the in each view a specification of the can box is done to capture the extremal motion state in all dimension. The three partial scan box specifications so obtained, one such specification per each view along respective axis of coordinate frames *X, Y* and *Z*, may then be combined geometrically for a complete spatial definition of scan box, and this is then dispatched to control interface CL. For each partial specification along respective standard axis *X, Y* or *Z*, again the user interface UI with the drawing tool may be used. A simple 2D displaying, eg on the existing display unit of imager's operating console OC, of the predicted surviews as stills or in in animation may be sufficient for most purposes. Alternatively, a 3D rendering such as based on the marching cubes algorithm or other is used, preferably with rotation of the target anatomical feature in whichever state, so that user can define the scan box perimeter in such 3D view at once for all three dimensions. The per dimension definition in partial specification may not necessarily be based on visualizations along standard axes $X,Y,Z$. Instead, such per view specifications of scan box SB may be based on reformatted surviews, and any new coordinate system with their axes $X',Y',Z'$ may be used instead of the standard coordinate system $X,Y,Z$.

A free drawing tool can be used in the user interface UI, or a guided such tool may be used which aides the user in defining a pre-set shape of the scan box. Such scan box is rendered based on user varying one or two dimensions thereof so as to allow the user to center the scan box accordingly, whilst the third dimension is adjusted and expanded dynamically to capture the extremal motion state (at maximal spatial deviation from initial motion state) in the appropriate predicted surview $Vj*$. Such guided drawing tool thus restrict user input along certain directions with auto-completion in others, to so respect the geometry (angles between edges, etc) of the pre-set scan box shape.

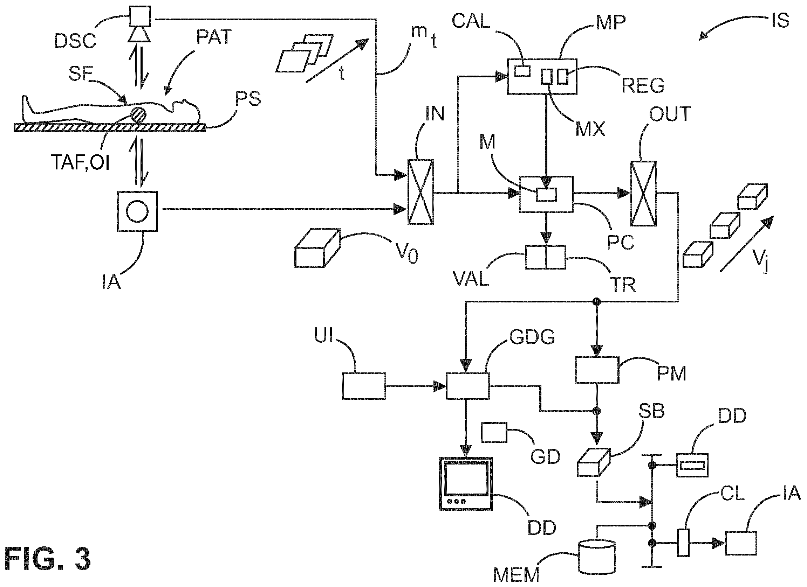

Reference is now made to Fig. 3, which shows a schematic block diagram of the facilitator systems FS as envisaged herein in embodiments.

Broadly, input data is received at input interface IN. Input data includes at least one surview V0. This is processed, optionally together with additional information supplied by a camera DSC (on which more below) by predictor component PC. Predictor component PC may use a motion model M to compute based on input data the one or more virtual surviews Vj which are made available for output through output interface OUT. Based on the output virtual surviews Vj, the scan box specification may be obtained and passed on to control interface for diagnostic/more detailed, but at any rate, higher IQ imaging, at an IQ higher than that of the input surview V0. The scan box SB specification may be automatically computed by planning module PM, or may be defined by user interface UI interaction with user as described above. In this case, planning module PM may still be used, in particular to translate the user input, such as drawing instructions as mentioned above, into the scan box SB specification. For example, the per view partial specifications may be combined by planning module PM into the required 3D specification of scan box SB.

The different virtual surviews Vj as output at external or internal output interface OUT are preferably of the same size (length, height, width) as the initial input surview V0. Planning module PM may combine the information from the virtual surviews Vj to compute the scan box SB, for example as a definition of a geometric shape such as the cuboid, ball, ellipsoid or any other, as mentioned earlier. The planning module in the automated embodiments may identify the extremal surview Vj * and fit a geometrical shape of predefined form to tightly over extremal points of the target anatomical feature as estimated in the extremal surview $Vj*$. Segmentation may be used with tangential fitting of the preset shape to so geometrically define the scan box. The scan box may be displayed, stored for later usage in a memory MEM; or may be passed onto the mentioned control interface CL in order to control subsequent operation in the diagnostic scan of the imager IA to acquire the second higher IQ projection data $\lambda$ in the diagnostic scan for the scan box voxels.

The so collected new projection data $\lambda$ at higher quality can then be reconstructed by reconstructor RECON into the diagnostic volume $V$ of the target anatomical feature at low noise and good contrast. This image may then be used for therapy, diagnosis, training or any other medical task for which it is needed. The above-described process flow may be referred to as the automatic one, as no user involvement is needed.

The second embodiment with user involvement via the user interface as described above is shown in the lower left of Fig. 3 where the graphics display generator GDG displays in a graphic display GD one or more of the virtual motion state surviews Vj, preferably in an motion picture /animation. Display of surview Vj on display device may be done selectively so that one or more of the predicted surviews Vj are brought to display, either singly or in conjunction with the original volume V0 and/or two or more of the virtual surviews Vj, Vj' are displayed as needed. Preferably, the animation is rendered by displaying the virtual surviews VJ preferably in combination with the original volume V0 in sequence one instead of the other in a given order. The order of display is preferably such as to respect the expected natural sequence of motion states, although this may not necessarily be required. Thus, the surviews Vj are either output by the predictor component in the correct order or output in any order and can then be re-ordered into the anatomical order by comparing the target anatomical feature positions as per the virtual surview Vj with the initial position as recorded in the initial input surview V0. Those virtual surviews Vj which are spatially closer to the original one are displayed first followed by those virtual surviews Vj that are spatially further away. Etc to so replicate a natural animation that respects anatomical realities. However, in terms of definition of the scan box, and display order may suffice, so long as the extremal surview(s) Vj* may be identified, either in 3D, or separately along each dimensional axis, as needed.

Referring now to embodiments of the facilitator system FS in more detail, and in terms of input data, two main embodiments are envisaged herein, as will be explained in more detail in the following.

One such embodiment relies solely on a single or multiple surviews V0 collected from prior measurements (projection data) collected in an exploratory image acquisition at low dose. The prediction of the virtual surviews Vj for other motion states of the target anatomical feature can then be done by the predictor component PC solely based on the single or multiple input surview volumes V0 received as input. In another embodiment however, which can be called a two-channel embodiment as opposed to the one channel embodiment just described, there is a second channel (the first channel being the one o more input surviews V0) in which video footage is acquired of a surrogate feature SF the patient PAT by a suitable non-ionizing radiation based camera DSC.

The footage camera DSC may be installed in the gantry SG of the imager IA. or in the bore BR to acquire such footage of the surrounding anatomy SF, such as of skin surface of the patient. However, such an arrangement within the bore or gantry is not necessarily needed it can also be installed at any point in the examination room in which the imager is installed so that the footage is acquired prior to the introduction of the patient into the bore. For example, the camera may be installed on a stand, on the wall or ceiling as needed. The camera DSC may be specially fitted or existing such cameras may reused for scan box planning. Such existing cameras may have been used for patient positioning or surveillance.

This over-time video footage $mt$ includes some dynamic information about how the motion state of the target anatomical feature changes. However, this information is not captured directly as the target anatomical feature is an internal anatomy and the camera DSC is non-ionizing. Instead the over-time video footage mt captured by camera relates to an external feature, a surrogate feature SF, such as patient's chest etc, that also undergoes motion state, but different to the motion states of the target anatomical feature of interest. However, motion states of the surrogate feature SF may correlate to the motion states of the target anatomical feature of interest, and such correlation may be harnessed herein in some such 2-channel embodiments.

The information from this surview image V0 and the video footage $m_t$ of camera DSC (which may include multiple frames $m_1$, $m_2$ and $m_3$, etc) are then combined and processed by predictor component PC, to compute the virtual surview Vj, based on which the scan box SB can be obtained by user and/or planning module PM. The camera is, as said, preferably operable based on non-ionizing radiation such as near infra-red (NIR), IR, or is based on the optical (visible) light spectrum. A depth sensing camera may be used that acquires a spatial profile of the surrogate feature SF such as chest and how its motion states change over time, with the respiratory cycle for example, which may be correlated to the motion states of the lung(s), or the heart, etc. The depth sensing image may be conceptualized as a 2D set of point measurements, with each pixel representing a depth measurement from a focal spot of camera DSC 's sensor along a respective geometrical ray that runs from focal spot to a respective point on a patient surface, such as point on patient skin or cloth covering the chest, with different pixels corresponding to different such rays and point on patient. Chest is one example for the surrogate feature SF whose motion states serve herein as an at least partial spatial information surrogate for the actual motion states of the target anatomical feature TAF of interest.

The depth sensing camera DSC, as one embodiment of the non-ionizing camera DSC, acquires a sequence of depth profiles from the reference position of the camera of the patient PAT's skin for example such as of patient's chest, etc. The depth values will change over time according to the motion of patient, in particular of the surrogate feature, path of skin, the chest, etc. Thus, the motion of the (internal) target anatomical feature which causes or is otherwise correlated with the observable motion states of the external surrogate feature within patient PAT cannot be directly observed with this approach as said. However, no additional dose is expended and it is instead the motion state of the surrogate anatomical feature SR, whose motion state is observed instead. For example, the surrogate feature SF (skin of chest) may sat least partly surround the target anatomical feature TAF (lung, heart) or may be otherwise spatially related. The motion of target anatomical feature TAF may or may not cause the motion of the anatomical feature, as is indeed the case for the lungs vs chest skin or heart beat vs chest skin, etc. However, such causality is not a necessity herein, so long as there is some correlation between motion (states) of target anatomical feature and surrogate feature SF.

Thus, spatial information about the motion of the surrogate feature SF, such as the patient's skin in the vicinity of the target anatomical feature, can be correlated and motion components may then be extracted from this information and applied to the original input surview V0 to so predict/extrapolate the virtual surviews Vj for other motion states of the target anatomical feature TAF. Again, a single such surview for the extremal position/motion state may suffice herein to defined scan box, but is preferred herein to acquire multiple such surviews, each representing different such intermediate motion states to obtain a more robust, realistic understanding of target anatomical feature's motion state. However, the extremal motion state should preferably be included also in the case where multiple such surviews Vj are computed. Again, more robust results may be achieved in this manner.

Turning now first to the two-channel embodiment in more detail, the predictor component PC may use a motion model M as will be described in more detail to compute the virtual surviews Vj for the different motion states of target anatomical feature TAF.

The motion model M as may be used by the predictor component PC for computing the virtual surviews Vj may be in either of two kinds. The model may be an explicit motion model or may be an implicit motion model. In the explicit motion model, motion components are explicitly computed as vectors, which may then be applied to the original surview image V0 to compute the predicted volumes Vj. For example, one single such vector may be used to translate the image representation of the target anatomical feature TAF as per the input surview V0. More than one such vector may be computed for different points across the three dimensions, and they may be applied at different points to the image representation m(TAF) of the target anatomical feature TAF to effect a deformation for example. Tensor or matrix representations may be used for more complex motion patterns. A rotation matrix may be used, etc. A segmentation may be used to geometrically define the said in-image image representation m(TAF) of target anatomical feature TAF. The one or more vector may be applied at points on the boundary of the in-image image representation m(TAF).

In another embodiment, in particular in the one-channel embodiment, a machine learning model is used such as an artificial neural network etc that does not require specific computing of motion components. In such a machine learning approach, the model may be able to extrapolate suitable patterns that have been learned from a large number of training data instances of input surview volumes V0 and known motion patterns whichever way obtained in order to learn the general relationship between input surview imagery V0, and the motion information. Thus, in this machine learning embodiment, based on even a single surview input image $V0$, a sequence of virtual surviews $Vj$ may be computed. In some embodiments, more than one surview image $V0$ may be applied as input. And as mentioned before, the machine learning model M may be configured to merely compute a single virtual surview, in particular the one $Vj^*$ for the extremal motion state.

In an alternative embodiment, machine learning model may be used in the two-channel approach. In this embodiment, the model processes video footage $m_t$ of patient PAT surface SF imagery acquired by the non-ionizing camera as explained above in combination with the surview input V0. The video footage captured the time evolution of the surrogate anatomical feature SF, such as a surrounding feature that may surround the target anatomical feature TAF. Such surrogate feature may include in particular surface of human skin, or any other body part whose motion can be correlated with the motion states of the internal target anatomical feature. The model M is trained training data to learn the relationship between surview imagery and such camera footage of surrogate feature SF. For each frame $m_{j,t=j}$, received from camera DSC, a corresponding virtual surview $Vj$ may computed that represents the motion state of the target anatomical feature TAF, given the initial information as per the measured input surview V0. Thus, conceptually, the surview input $V0$ may be understood as a data based regularizer for model M, whilst the surface image stream by camera provides the motion information. Thus, the combined information of input surview $V0$ and surface video stream $m_{j,t=j}$ help "steer" model M to provide the "right" virtual surview $Vj$ for each or some footage frame instant j .

Turning first to the explicit motion modelling embodiment in more detail, the facilitator FS may include various one or more components such as a calibrator CAL, a registration unit REG and/or a motion component extractor MX. Such components may be part of a motion model adaptor MP, operable to adapt motion model M to the specifics of the given patient PAT and the current imaging setup in including the imaging geometry.

Broadly the calibrator CAL calibrates the acquired camera footage $m_t$ with the imaging geometry. Registration unit REG registers the footage $m_t$ with the chosen motion model M. In more detail, the calibrator calibrates the camera images with the imaging device's imaging geometry. The camera and CT scanner are typically installed at fixed position in the scanner room and their respective distances, angulations and other geometry parameters are known from the system specifications. Additionally, a registration procedure by registration unit REG can be performed during installation and may optionally be verified at regular intervals. For example, markers placed at known positions at the patient couch PS or elsewhere can be identified in the camera RGB images and used for this purpose. Other techniques can be employed.

The explicit motion modelling may include extraction by motion extractor MX of motion vectors from the motion footage $m_t$ The extracted motion information may then be registered by registration unit REG with the timing of the video footage $m_t$. Once the motion components (vector(s), matrix, tensor, etc) are suitably registered time- and/or space-wise, they can then be applied to the surview input V0 that serves as part of the model in this case. For example, a vector field may be applied to expand or compress or otherwise move, such as rotate, translate, etc, voxel of surview image V0. This allows extrapolating the different motion states. Thus, virtual surviews Vj may be extrapolated, one for each new video frame mt, with corresponding motion vector $\vec{v_t} = \vec{v}(m_t)$ extracted therefrom. At this point, all voxels of surview V0 may be subjected to application of the extracted motion $\vec{v_t}$, including the voxels that make up the in-image representation m(TAF) of the target anatomical feature TAF. However, focusing application of motion information $\vec{v_t}$ only on the in-image representation $m(TAF)$ of the target anatomical feature TAF can still be done by using a segmentor (not shown). However, for this, a suitably trained segmentor may be needed that is able to cope with the low signal, high noise data as is the low dose surview image V0. Machine learning based segmentors can be used, trained on ground truth data that may include artificial noise corrupted training input imagery. A similarly trained segmentor may also be used by the planning model PM when computing fitting scan box shape around such a segmentation $s(m(TAF))$ of the in-image representation $m(TAF)$ of the target anatomical feature TAF.

In addition, or instead, a segmentor so trained may be used in the user interface UI based embodiment, to better assist the user in being able to draw the scan box sufficiently tight around the segmented structure $s(m(TAF)$. As to the tightness requirement, a dead space $\Delta = 0$ is preferred herein, but this may not be necessarily practiced in all embodiments, and a more relaxed dead space requirement $\Delta'$ with some suitably small error margin may be acceptable, $\Delta' > 0$.

As an optional feature, the system FS may include a motion validator VAL. The motion validator VAL may compare extracted motion with mode predicted motion. If there is a deviation in excess of a preset threshold, there may have been abnormal patient motion. This can be flagged, and a control signal may be passed to a transducer TR (such as a display device, speaker, lamp, etc). The transducer TR may respond with issuing a sensor alert signal to alert user about the possibly abnormal patient motion that may be detrimental to IQ.

Operation of some of the components of facilitator FS are now described in yet more detail in relation to the explicit motion modelling embodiment, as opposed to implicit modelling (machine learning based) as will described later below. The explicit motion modeling is preferably of the motion of surrogate feature SF (surface of patient, such as skin surface of chest, etc). The modelled motion of surrogate feature SF may be known to be correlated to the motion of the target anatomical feature TAF.

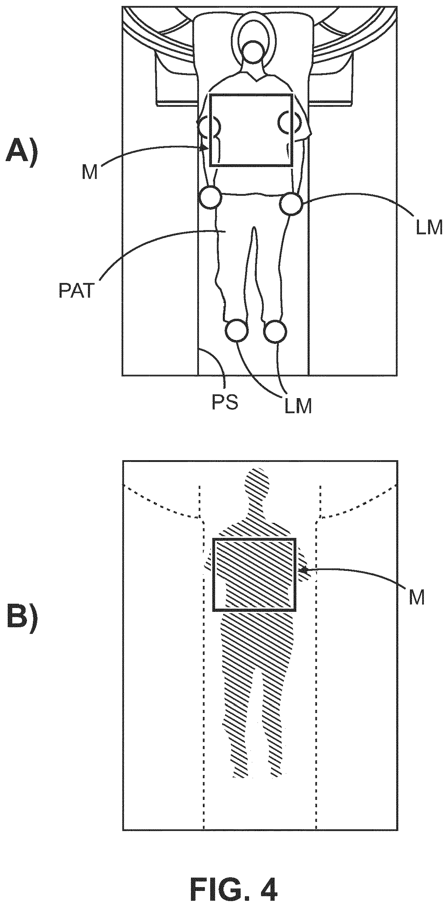

Fig. 4 is an illustration of operation of the registration unit REG and the calibrator CAL for explicit motion modelling. As an example, respiratory cycle-based modelling is used, but the principles described herein are readily applicable to other scenarios.

In general, and as mentioned earlier register the motion model in space and time is preferred herein. Space-wise registration may be understood to match which part of the moving portion in the surview corresponds to the motion recorded with the camera, eg, depth sensing camera. Timewise registration may be understood as identification of phase information, such as inhale and exhale or heart phase etc. The latter, time-wise registration may be achieved by telling patient to exhale or inhale before the surview is taken.

Turning now in more detail to space-wise registration, the actual patient position on the couch may be registered by registration unit REG with the camera images $m_t$. Thus, a registration-based method can be used for this purpose. For example, certain landmarks LM of the patient identified in the RGB-channel of video stream (such as eyes, mouth, hands, joints, among others) can be used to identify the position of the patient on or relative to the couch PS. These landmarks LM can be used to spatially align patient PAT with the explicit motion model M. Depending on the surrogate feature SF or the target anatomical feature TAF, a respiration prediction model may be used. It is preferable to know which 2D region of the D-channel (depth-channel) of video stream mt should be used to observe the distance changes induced by the respiration, as one example of surrogate feature SF. Thus, within the footage a target region may be identified that represents motion of surrogate feature SF.

A patient specific breathing motion model may be selected or generated. Model preparer MP may be operable to calibrated / parameterize model M with the actual breathing cycle of patient over time, or whatever the surrogate feature SF. For example, the D-channel of the camera and the target region may be used. The minimal and maximal distance of the patient surface SF within the breathing cycle is identified and used for the calibration of the motion model in time domain. Once the model M is so prepared, motion extractor MX extracts motion component. Predictor component may apply this motion component to some or each voxel of the 3D surview V0, and for each or each point in time. For example, a translation of target anatomical feature TAF voxels due to respiration may be calculated using the explicit model to obtain a virtual surview $Vj$ for a given time $t = j$. This may be repeated over time, to generate the sequence $Vj$ which can then be displayed as animation to confer the extent of the motion, thereby facilitating definition of tight scan box that covers target anatomical feature TAF in most, if not all, motion states.

With continued reference to Fig. 4, and providing more detail on the model preparer MP in the context of explicit motion modeling, the said patient landmarks may be identified with the RGB camera DSC to spatially align the 3D respiration model with the patient, specifically, to align the target region to be observed in the D-channel of the camera for the calculation of the distance changes due to the respiration of the patient. Fig. 4 illustrates this approach in relation to the target region. The rectangle as shown in Fig. 4, illustrates at least a part of the model M. The rectangle illustrates the spatial alignment of the region (the "target region") in the D-channel based on the correlation with the landmarks LM as identified in the RGB-channel of the camera. For example, posterior position is aligned with the patient table PS. The target region is the motion information in the footage that represent the motion states of the surrogate feature SF. Thus, spatial registration of the motion model (illustrated by the rectangle in Fig. 4) may be based on landmarks identified in the RGB-channel of the camera. Only pixels in the rectangle are used to determine the temporal breathing state. The breathing state uses values of the D-channel. Use of a RGB-D camera that combines optical imagery and depth information is not a necessity herein. Both channels may be provided by separate cameras instead. The optical channel/optical camera allows identifying, using image recognition techniques, the landmarks, whilst the depth channel/depth camera allows recoding the motion state information of the surrogate feature SF. Again, the optical channel is optional and may not be needed in some embodiments.

Instead of using correlation with landmarks LM, the input 3D surview $V0$ itself can be used instead for the registration. The input 3D image $V0$ provides some tissue and bone structures, which can be registered with a 3D atlas of the human body, which itself can be "elastically" matched using the outline of the patient (as may be identified in the RGB-channel) and the outline of the atlas.

In another embodiment, an exact spatial alignment of the landmarks LM and motion model between the camera data and the surview data may not be necessary. Instead, a temporal motion model may be extracted from the camera data and is directly applied to the surview data V0 to generate the breathing induced deformations.

Temporal alignment of the 3D respiration model may be based on an identification of the inhale and exhale motion states. Such inhale and exhale motion states are examples of the mentioned extremal motion states, which are not only present for target anatomical feature TAF but may also be manifest in the correlatable motion states of the surrogate feature SF. Preferably, for robustness, some intermediate motion states are also identified.

There are several options for the selection of a suitable breathing model, depending on which surrogate (anatomical) feature SF is used. For motion state changes of such surrogate feature SF due to respiration the following options may be considered.

One such model may allow for movements in anterior / posterior direction and a linear interpolation between the maximum and minimum inhale / exhale state (anterior) may be identified in the D-channel in the target region. The posterior position is fixed.

Such model may be refined by automatically segmenting the 3D surview VO in bone and soft-tissue structures. The interpolation can then distinguish between rigid and more elastic moving parts in the breathing cycle.

The model is preferably spatially aligned with the 3D surview V0, and matched with the breathing cycle. Thus, it may be beneficial to record in which motion state (inhale / exhale) the 3D surview VO was acquired as mentioned above. This matching can be easily achieved when the camera detects the breathing motion also during the acquisition of the surview for example.

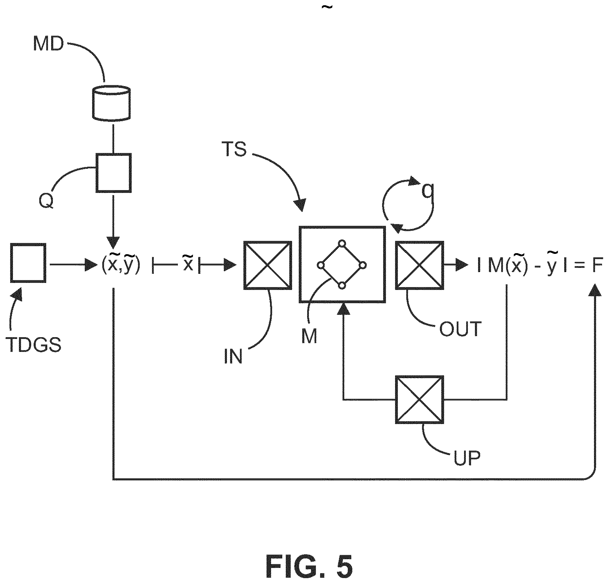

In another embodiment, and now turning to the machine learning model based implicit motion modelling in more detail, various approaches are envisaged herein, either in the two-channel embodiment (where input data include camera footage $m_t$ and input surview $V0$), or in the one-channel embodiment (where input data includes surview V0 without camera footage $m_t$). Such ML approaches may use training data. A training system TS as schematically shown in Fig. 5 may use a machine learning algorithm to adjust parameters $\theta$ of model M, based on such training data $\{(\tilde{x},\tilde{y})\}$. Machine learning models may be generic and may not require explicit analytical modeling of motion components as explained above in the explicit motion modelling-based embodiments.

For example, in one embodiment, a query facility Q is used to locate in medical databases MD such as HIS (hospital information system) or PACS (picture archiving and communication system) historical patient records of various patients suitably varied in age and gender and across demographics for those cases where at one point in time a surview image including the anatomy of interest TAF was acquired.

Some imaging setups used in such historical exams may have included such a video footage camera DSC as mentioned above, suitable installed in exam room, at gantry or withing bore as mentioned earlier. Such camera DSC may have acquired footage of surrounding anatomy SF, such as the skin surface of the patient during acquisition of the projection data for the surview V0. Such cameras may have been used for other purposes (such as patient surveillance, etc) than intended herein. However, such imagery me be reused for the present objective of training machine learning model M for predicting virtual surviews $Vj$.

By acquiring such camera footage of surrogate feature SF such as of chest skin, etc, during the acquisition of the projection data for the input surview image V0, naturally associated pairs of training input data (the video footage) and their targets (the respective surviews $V0$) can be collected. The reconstructed surviews $V0$ can thus be associated using time stamps with certain surface images such as depth sensing profile acquired during the projection data acquisition. Such instants $i$ of pairs of training data $(\tilde{x}=m_t, \tilde{y}=V0)^i$ can then be fed as training data into the ML model M for training. The model M may thus be trained to associate surface imagery of the surrounding structure SF of different patients with different associated instances of surviews V0. Thus, the dynamical correlation between such video footage and the corresponding surview imagery $V0$ may be learned. It may be difficult to locate such pairs of surface footage and associated surviews among historical patient records. In such cases, an experimental data collection project at different medical facilitates may be setup. Such project may include installing cameras at tomographic imaging equipment such as CT or MRI, etc, and to so acquire over time a large enough stockpile of pairs of such training data. Alternatively, existing in-bore/in-gantry cameras originally intended for patient surveillance during imaging may be so repurposed.

Thus, in this 2-channel ML approach, correspondence between temporal motion state measured by camera for surrogate feature SF (such as at the surface of the thorax) and the internal organ motion state of target anatomical feature TAF as per surview V0, can be learned from training data. The training data may include the said pairs of multiple 2D or 3D tomographic images (MRI, CT, US) acquired during breathing and related camera footage.

A regression type machine learning model, such as a neural network model, preferably of the convolutional type, may be used to learn this relationship between the surface imagery and associated surview. Once trained, and in deployment/inference phase, the machine learning model takes as input a current surview and a currently acquired surface video frame to compute the virtual surview for the given video frame and initial (projection measurement based) surview V0. Thus, the above is an example for a two-channel application with machine learning implementation.

In another approach, generative machine learning modelling is used. Such approach may allow setting up a one-channel where only the input surview V0 is needed. No camera DSC is required herein, but may still be used, if needed. In this generative ML modelling approach, database querier Q is used to locate historical imagery including different kinds of surview imagery, that captured the anatomy of interest for various patients. It is unlikely that all these surview images have been collected in the same motion state. Thus, the motion states encoded by those historical surviews V0 may encode different motion states thus effectively providing samples of surview footages across different demographics. This training footage may be sufficient for a generative type ML network to learn virtual surview footages Vj, given any input initial surview image V0. Then, after training, in deployment, the generative model may be repeatedly applied, starting from the input surview $M(... M(M(V0)) ... ) = Vj$ (with index j being the number times Model M is iteratively applied) to generate different instances of virtual surviews Vj at different motion states of the target anatomical feature TAF. Alternatively, the model may be trained to output a sequence of predicted surviews given input surview. For example, historical surviews v0 (written in lower case) may be spilt up into input $\tilde{x}=v0j$ for any given instance j, and two or more of the historical surviues may be used as target $\tilde{y} = v0^k, v0^{k+1}, ... v0^{k+m}$. This can be split up into different such combinations to form a decent sized training data set. *"m"* determines the "length" of the sequence (and hence of the animation, if needed) of surviews one wishes to predict.

Generative ML modelling may be conceptualized as a sampling from an underlying probability distribution of the space from which the training data was drawn.

Machine learning models of the generative type may include those of the GAN-type(generative adversarial networks), variational autoencoders (VAEs), mixture models such as Hidden Markov Models (HMMs), Markov Random Fields (MRF), etc. Some such generative models may be based on ANNs (artificial neural network models), preferably of the convolution type (CNNs) as such models have been observed to yield good results in spatially correlated data as are images such as the surview imagery of main interest herein. As an extension or as alternative to CNN models, Transformer type models may be used, with attention mechanisms where convolutions filters are adapted to act differently on different portions of intermediate feature map inputs.

Turning now to the embodiments of GAN type ML models M, such ML model types have been previously described by Ian Goodfellow et al in "Generative Adversarial Nets", available on preprint server at arXiv: 1406.2661v1, published 10 Jun 2014.

GAN type learning may comprise two NN models, a discriminator network ("D") and a generator network G. The discriminator D may be set-up as an NN-classifier. The discriminator D may be arranged as a deep fully connect network, a deep fully convolutional network, or as a hybrid network including one or more layers of both type.

In operation during training, the GAN set-up works as follows. The generator G generates from a seed signal (r) applied to it (such as random noise for example), a sample surview G(r). The discriminator D attempts to discriminate between the generator output G(r), and genuine, surview sequences , as may held in a training database. The ground truth comprises historic sequences of surviews V0$^i$ that represent the target anatomical feature TAF at different motion states for different patients, collected from medical databases by querying as described above.

A specially configured cost function may be used, based on which the parameters of generator G and discriminator D are adjusted. Such as binary cross-entropy cost function was described by *Goodfellow et al* (*supra*) at their eq(1), page 3. The cost function represents two opposed objectives similar to a zero-sum-game: the objective in respect of the discriminator D is to maximize its probability of correct labelling, whilst the objective in respect of generator G is produce output so that the discriminator D cannot statistically discriminate between a real surview V0 drawn from the training data, and generated virtual surviews generated by the generator.

The parameters of the two models G,M may be adjusted in a min-max procedure in alternate manner in separate runs of iterations. The iterations cause the parameters of the models G,D to converge in a type of a Nash-equilibrium, at which point the generator is capable of generating realistic looking "fake surviews". At this point, the discriminator D is no longer capable for discriminating between fake surviews and ground truth, and the discriminator D is such no longer needed. The generator G can thus be used to generate from a given surview V0, virtual surviews.

As an extension of GAN-type generative ML models, conditional GANs (cGANS) may be used.

However, generative modelling is not the only option for one-channel embodiments. In addition, any regression type ML model setups configured for processing of sequential type data may be envisaged herein such as recurrent NNs. A type of recurrent NNs may include LSTM (long short-term memory networks,), etc.

Reference is now made in more detail to the training system TS in Fig. 5. Broadly, in relation to ML models, three processing phases may considered: a prior training/learning phase, an optional subsequent testing phase, and thereafter, the deployment/inference phase. In training phase parameter of model are adjusted based on the training data set. A training/learning algorithm may be used to effect the training. In testing performance of model is checked based on test data. In deployment/inference phase is used in the intended environment such as in clinical practice when deployment data is processed by model to compute the virtual surviews. Test data is different from training data. And the deployment data is different from both, test and training data. Training system TS implements the training phase. Training phase may be a one-off, or may be done repeatedly using the earlier trained version of model as starting point. For example, training may be repeated once new training data becomes available.

The training system TS may be implemented on one or more computing systems, and is configured for training the ML model M. The training data is shown as pairs $(\tilde{x}, \tilde{y})^k$ for a supervised setting, with $\tilde{x}$ indicating a training input image, and $\tilde{y}$ its associated target and index "L" an index for a training pair, the training pairs making up the training data set.

In training phase, an architecture of the machine learning model M, such as a CNN, is pre-populated with initial set of weights. The weights $\theta$ of the model NN represent a parameterization $M^\theta$. The weights may include filter parameters of the convolutional operators CV. It is the object of the training system TS to optimize and hence adapt the parameters $\theta$ based on the training data $(\tilde{x}_k, \tilde{y}_k)^k$ pairs. In other words, the learning can be formulized mathematically as an optimization scheme where a cost function *F* is minimized although the dual formulation of maximizing a utility function may be used instead. Instead of such an initialized model, a pre-trained model may be used.

Assuming for now the paradigm of a cost function *F*, this measures the aggregated residue(s), that is, the summed error incurred between data estimated by the neural network model NN and the targets as per some, or all, of the training data pairs k in a batch or over all training data:

$$argmin_\theta F = \sum_k \mathrm{D}\left[M^\theta(\tilde{y}_k), \tilde{y}_k\right] \tag{1}$$

In eq. (1), function *M*() denotes the result of the model M applied to training input $\tilde{x}$. The result will differ in general from the associated target $\tilde{y}$. This difference, or the respective residuals for each training pair i, are measured by a distance measure D[-,-] Thus, the cost function *F* may be pixel/voxel-based, such as the L1 or L2-norm cost function, or any other norm Lp. Specifically, The Euclidean-type cost function in (1) (such as least squares or similar) may be used for the above mentioned regression task when output layer regresses into the one or more virtual surviews. The cost function in (1) is merely exemplary. Other cost functions may be used such as the cross-entropy based cost function for the generator G and Discriminator D in the GAN model or cGAN model as mentioned earlier.

The output training data $M(\tilde{x}_k)$ is an estimate for target $\tilde{y}_k$ associated with the applied input training image data $\tilde{x}_k$. As mentioned, in general there is an error between this output $M(\tilde{x}_k)$ and the associated target $\tilde{y}_k$ for each pair *k*. An optimization procedure such as backward/forward propagation or other gradient based method may then be used to adapt the parameters $\theta$ of the model M so as to decrease the residue for the considered pair $(\tilde{x}_k, \tilde{y}_k)$ or, preferably for a sum of residues in batch (a subset) of training pairs from the full training data set.

The optimization procedure may proceed iteratively. After one or more iterations in a first, inner, loop in which the parameters $\theta$ of the model are updated by updater UP for the current batch of pairs $(\tilde{x}_k, y_k)$, the training system TS enters a second, an outer, loop where a next training data pair $\tilde{x}^{k+1}$, $\tilde{y}^{k+1}$ or a next batch is processed accordingly. The structure of updater UP depends on the optimization procedure used. For example, the inner loop as administered by updater UP may be implemented by one or more forward and backward passes in a forward/backpropagation algorithm or other gradient based setup, based on the gradient of *F*. While adapting the parameters, the aggregated, for example summed, residues of all the training pairs are considered. The aggregated residue can be formed by configuring the objective function *F* as a sum of squared residues such as in eq. (1) of some or all considered residues for each batch. Other algebraic combinations instead of sums of squares are also envisaged. In general, the outer loop passes over batches (sets) of training data items. Each set ("batch") comprising plural training data items and the summation in (1) extends over the whole respective batch, rather than iterating one by one through the training pairs, although this latter option is not excluded herein.

Optionally, one or more batch normalization operators (not shown) may be used. The batch normalization operators may be integrated into the model M, for example coupled to one or more of the convolutional operator CV in a layer. BN operators allow mitigating vanishing gradient effects, the gradual reduction of gradient magnitude in the repeated forward and backward passes experienced during gradient-based learning algorithms in the learning phase of the model M The batch normalization operators BN may be used in training, but may also be used in deployment.

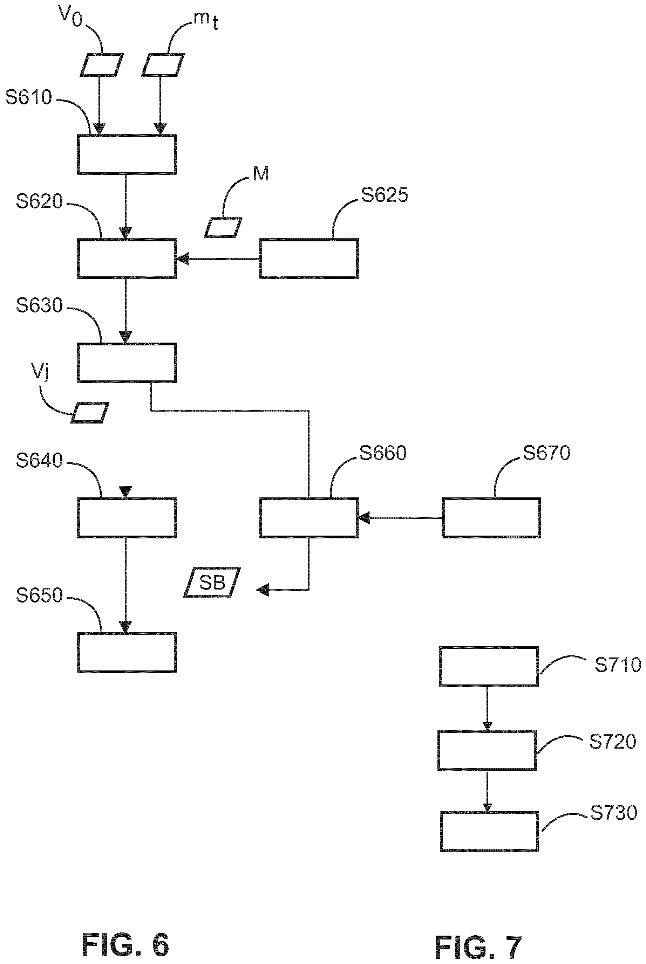

The training system as shown in Fig. 7 can be considered for all learning schemes, in particular supervised schemes. Unsupervised learning schemes may also be envisaged herein in alternative embodiments. GPU(s) maybe used to implement the training system TS. The fully trained machine learning module M may be stored in one or more memories MEM, and can be made available as trained machine learning models for use in system FS.

In addition, or instead of, sourcing historical surview imagery, the training data may be artificially generated by a training data generator system TDGS. For example, generative models may be used, such as GANs, that can be trained on some ground truth data to produce realistic looking surface footage representing the right motion state of the surrogate feature SF of interest. Historical surviews may be easier sourced from existing stock. The so generated footages can then be paired up with the respective surview to obtain training data set *{(x,y)}*.

ML models as envisaged herein may also use parameterized statistical models.

For example, from MRI, a series of base-line 3D surviews of a volunteer are acquired at different motion states over the breathing cycle. By using image registration methodology, a patient-specific breathing model can be derived from the base line volunteer imagery, and generalized using other such volunteer studies. In this manner, a parameterized, statistical model may be obtained.

Reference is now made to Fig. 6 which shows a flow chart of a computer implemented method of facilitating the collection of planning data, in particular predicting a scan box based on a surview volume. The planning data may be used to control projection data acquisition in a tomographic imaging.

At step S610 input data is received. The input data may include a 3D surview volume. The input surview V0 includes a representation of a target anatomical feature or of an object of interest OI. The surview has been reconstructed from low dosage projection data previously collected in an exploratory image acquisition by the imaging apparatus. Thus, the input surview is based on measurements for such are the collected low dosage projection data. The field of view (FOV) when collecting the low dosage projection data is large (not tight) and covers a wider anatomy than the target anatomical feature TAF. In particular, the FOV is not tight as the target anatomical feature TAF may be subject to motion. In extreme cases, the surview V0 may be based on a whole-body scan, or on scanning a wider anatomical section such as the whole torso, even if the target anatomical feature TAF is only one of lung(s), heart, or abdominal structures for example.

Usually, the surview covers a large field-of-view ranging from neck to pelvis bone. The surview is a static image acquired before the actual diagnostic procedure. Right before the surview is acquired, the patient is asked to hold her / his breath. Hence, the respiration state of the 3D surview is known and in this information be used in the following processing.

In some embodiments, the input data is multi-channel and includes in addition to the input surview V0, video camera footage collected of an external part of the patient, referred to herein as the surrogate feature SF. The camera used to record the footage is based on non-ionizing radiation such as optical camera, IR, NIR, LIDAR. A depth sensing camera such as RGB-D may be used to collect over time depth profiles of the surrogate feature SF. The surrogate feature SF may be subject to motion which may be correlatable motion of the internal target anatomical feature TAF. Thus, the video footage records such motion of the surrogate feature SF over time. Surrogate feature SF may include for example a patient surface such as skin, at least partly surrounding the anatomy of interest TAF. The over-time length of the footage is so that all the expected motion cycles of the target anatomical feature TAF / surrogate feature SF are recorded, in case of periodic motion. But even for non-periodic motion, footage should be recorded over a sufficiently long period for better robustness to so ensure enough motion information is collected. The exact length of footage will depend on the case at hand. The camera may be mounted in the scanner room, typically at a position where the camera can observe the couch PS and patient. The footage may record parts or the whole of patient over time.

At step S620 the input data is processed based preferrably on a motion model M to compute one or more virtual predicted surview images that represents the target anatomy at different motion states than the motion state represented by the input surview $V0$. As said, it is technically possible to output a single such volume that represents an extremal motion state having the furthest spatial reach as compared to the motion state of the target anatomical feature TAF captured by the input surview. Preferably however, multiple virtual surviews $V0$ are computed, including those that represent one or more intermediate motion states in between the initial motion state as per initial surview $V0$ and extremal motion state as per extremal surview $V_{j*}$.

If a two-channel approach is used as mentioned above, step S620 may include extracting motion component(s), such as in vector, matrix or tensor form, from the video footage $m_t$ acquired by camera of the surrogate feature SF. The one or more extracted motion components can be applied to the initial volume $V0,$ eg, to effect translation, rotation deformation (deformation or compression such in heart motion), as needed, to simulate the different motion states to so obtain different predicted virtual volumes $V_j$. Thus, this embodiment is an example of explicit motion modelling. Such embodiment may include a motion model setup phase, including calibration in respect of footage and imager IA's geometry/surview V0. The model preparation may also include registration, segmentation, etc.

In some embodiments, a single-channel approach is used where it is only the surview input image V0 that is fed into the model to prediction of the one or more virtual surviews Vj representing the different motion states, different from the one in the initial volume. In this single-channel approach, implicit modelling such as a machine learning model, may be used. The model may be of the generative type to predict the sequence of virtual surviews Vj. The modelling may be based on NNs, in particular CNNs. Recurrent models may be used, such as LSTMs or others. The model preparation in this case includes using an ML machine learning algorithm so adjust parameters of the ML model based on training data.

The two-channel approach using input surview V0 and surface footage $m_t$ may also be used with machine learning models as described above.

Computing of the virtual surviews may be done as a sequence at once or may be timed with the timing of the footage acquisition. Thus, at some or each instance $t$ of video frame $m_t$, a corresponding virtual surview is computed $m_{t,\ t=j} \rightarrow Vj$. Thus, there may be some latency until all virtual surview $Vj$ are computed over a sufficient length of time. This per video frame computing of the virtual surview $Vj$ may be used in explicit modeling or in implicit modelling (ML based modelling). Information from more than one video frame may be used to compute any given virtual surview Vj.

In an optional step (not shown), the motion of surrogate feature as extracted from the footage is compared to the motion model. A deviation between the model prediction and the actual recorded footage may observed in a validation step. If such deviation exceeds a threshold, this fact may be flagged up as an anomaly as this may indicate an unexpected movement of the patient. An alert may be issued to user. A transducer TR, such as visual, haptic, acoustic or any other may be used to issue the alert. Thus, the collection of footage may continue during the image acquisition for the diagnostic scan to serve as a motion guardian against unintended patient motion which may cause motion artifacts.

At step S630 the predicted virtual surviews Vj are then output and made available for further processing.

For example, at step S640 a scan box is computed based on the one or more projected surviews Vj. At a tight boundary surface of any given geometrical shape or irregular shape such as a convex structure is formed to include all the different portions as occupied by the target anatomy of interest as represented in the predicted surviews VJ.

The scan box may be so computed automatically. The scan box may then be further processed, such as stored, or may be used for controlling at step S650 a diagnostic acquisition.

The scan box computed herein is patient-specific scan-box takes into consideration the relative patient motion.

The computed scan box can be displayed in addition to a scan-box already defined manually by the technician at the console.

In another embodiment, the predicted virtual surviews Vj are displayed at step S660 in sequence as an animation. Alternatively, or in addition, after or before replay of such animation, selected one or more of the virtual surviews Vj are displayed, optionally in combination with the input initial surview V0, as needed. Based on displaying the virtual surview Vj as one or more still or in over-time animation (video), the user can then use a graphical user interface or any other user interface at step S670 to demark a scan box based on the displayed surviews V0, Vj.

At step S660 the animation over virtual surviews Vj may be replayed a moving picture that illustrates transitioning between the different motion states of the target anatomical feature TAF. The user can then easily ascertain the extremal motion state with the furthest positional deviation as compared to the motion state recorded in the initial input image V0 and this can then be used to define a tight scan box demarcation in one or more spatial views. The boundary surface of the scan box may be chosen tangentionally from plural views to osculate the target anatomical feature's surface as represented in its extremal motion state surview Vj *. This will then define a scan box in a tight manner to ensure that it includes all motion states, but not any irrelevant voxels which the target anatomical feature TAF never visits. This allows saving dose and allows procuring good image quality.

The above-described definition of the scan box may be done in different views one after the other in X, Y and Z direction and this is then combined to ensure that a motion in different, eg all, spatial dimensions are accounted for.

Thus, steps S660, 670 represent a user-input based manner of defining the scan box, whilst in the fully automatic embodiment step S640 the scan box is computed without such displaying based on the virtual surview Vj's. Segmentation for the various in-image representations $m$(TAF) of target anatomical feature TAF may be used in the manual and/or automatic embodiment.

At step S640, even if scan box is computed automatically, the virtual surviews Vj, either at stills or as animation may be displayed as additional information. A visualization of the computed scan box may be displayed, either on its own, or, preferably, in combination with initial surview *V0* or virtual surview *Vj.* For example, scan box may be visualized as an overlay or 3D rendered on initial surview *V0,* or on any one or more of the virtual surviews *Vj,* in particular on the extremal virtual surview *Vj\*.* The various manners of visualizations and displaying are preferably used in conjunction with the user input-based embodiment as per steps S660, S670. However, such may also be used in the automated embodiment where the scan box is calculated based on the predicted virtual surviews *Vj.*

Reference is now made to Fig. 7 which shows a flow chart of the machine learning model based embodiment (implicit motion modelling). In such models the motion information is learned implicitly from patterns in training data, rather than by explicit, analytical modelling.

At step S710 training data is obtained.

At step S720 a machine learning model is adapted based on the training data and at step S730 the so trained model is made available for deployment or testing, such as in in clinical practice. The input surview *V0,* and optionally the footage, encountered during deployment are different from the training data.

The obtaining S710 of the training data may be done synthetically by generative machine learning models or may be done by querying existing medical data records. The latter may include querying for patients whose records include respective surviews $\bar{x}$=V0, large enough to include the target anatomical feature of interest. Preferably such historical record may further include the mentioned footage of a suitable surrogate feature of patient that is correlatable to motion of the target anatomical feature.

In yet another embodiment the obtaining of the training data may include installing a surface camera within the bore of the tomographic imaging apparatus to acquire alongside the projection data for the surview image *V0* corresponding surface imagery for example of the patient's chest or any other body part.

The so collected footage may then be associated with different instances of surview imagery V0 across the patient.

In yet another embodiment, surview images from different patients are collected under the assumption that some or each represent a different motion state. Any one of the so collected surviews may then be used as training input with the remaining one, two or more representing the target sequence which can then be used to train a model of the generative type, preferably conditionable, such as cGAN, based on the input surview *V0.*

Generative type ML models include GANS, preferably conditionable, such as cGAN, VAE and others.

Supervised training scheme with labelled data, or unsupervised, or self-supervised training schemes may be used.

Any of the above-described system FS and methods may be of equal application to other tomographic imaging modalities where tomographic raw data (eg, projection data) needs or be acquired, such as in MRI, SPECT/PET or other nuclear imaging modalities. In such other than CT modalities, a first low IQ scan is used, based on which imaging planning data (such as the scan box) is derived, and this is then used to control the second, higher IQ scan. The low IQ scan may be for a larger volume/areaIFOV than the volume/area/FOIV for the later, high(er) IQ scan. For example, in MRI, the surview *V0* there may correspond to a faster scan than in the diagnostic scan, with lower resolution, thicker slices, lower signal-to-noise ratio, but larger FOV, to so obtain the overview needed for planning the diagnostic scan.

Components of the facilitator system FS may be implemented as one or more software modules, run on one or more general-purpose processing units PU such as a workstation associated with the imager facilitator system FS, or on a server computer associated with a group of imagers.

Alternatively, some or all components of the facilitator system FS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the facilitator system FS may be implemented in both, partly in software and partly in hardware.

Different components of the facilitator system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs/characters used in the claims should not be construed as limiting the scope.

## EP 4 491 125 A1

**Claims**

1.  System (FS) for facilitating planning of raw data acquisition by a tomographic imaging apparatus (IA), comprising:

    an input interface (IN) through which is receivable an input 3D surview image ($V0$) of a movable object (OI, TAF) occupying a first portion of 3D space, the surview image ($V0$) previously obtained by the, or a, tomographic imaging apparatus based on raw data acquired at a lower quality than a quality intended for the planned raw data acquisition, and the surview image representative of a first motion state of the object motion;
    a predictor component (PC) capable of predicting, based at least on the input surview image($V0$), one or more predicted 3D surview images ($Vj, j>0$) representable of the object in other one or more predicted motion states, and the object in the one or more predicted motion states occupying one or more other portions of space, and
    an output interface (OUT) capable of providing at least the one or more predicted surview images ($Vj$) for the said facilitating of the said planned raw data acquisition.

2.  System of claim 1, comprising a planner module (PM) capable of computing planning data based on the predicted one or more 3D surview images ($Vj$).

3.  System of claim 1 or 2, wherein the planning data includes a specification of a target portion (SB) of the 3D space that includes the one or more other portions of space, and wherein the target portion of space is a subset of the input surview image ($V0$).

4.  System of any one of the preceding claims, the system comprising a graphics display generator (GDG) capable for generating a graphics display (GD) for displaying on a display device (DD), the generated graphics display (GD) capable of visualizing the one of more of the different motion states of the object based on the one or more predicted 3D surview images ($Vj$).

5.  System of any one of the preceding claims, wherein i) the graphics display (GD) includes video capable of representing an animation of the object transitioning between the different motion states, thereby occupying the different portions of space, and/or ii) wherein the system comprises a user interface (UI) capable to allow a user to designate the target portion in space based on the graphics display.

6.  System of any one of the preceding claims, wherein the predictor component (PC) capable of computing the one or more predicted 3D surview images based on i) additional input data, including video footage acquirable of a surrogate feature (SF) in relation to the object by a non-ionizing radiation based camera (DSC), and/or ii) a motion model (M) for the said object motion.

7.  System of claim 6, wherein the motion model (M) is a trained machine learning model trained on training data.

8.  System of claim 6, wherein the motion model (M) represents at least one motion component of the motion as extractable from the video footage and wherein the predictor component (PC) to apply the so extracted motion component to the surview image ($V0$) to so extrapolate the predicted 3D surview images (Vj).

9.  System of any one of the preceding claims, including a transducer (TR) capable of issuing an alert signal if the video footage of the non-ionizing radiation based camera is indicative of a deviation between the so imaged motion state and the model, and wherein the deviation is in violation of a pre-defined acceptability condition.

10. An imaging arrangement (MAR) including the system of any one or more of the preceding claims, and any one or more of: the tomographic imager, the camera, the display device.

11. Computing-implemented method of facilitating planning of raw data acquisition by a tomographic imaging apparatus, comprising

    receiving (S610) an input 3D surview image (V0) of a movable object (OI) occupying a first portion of 3D space, the surview image (V0) previously obtained by the, or a, tomographic imaging apparatus based on raw data acquired at a lower quality than a quality intended for the planned raw data acquisition, and the surview image representative of a first motion state of the object motion;
    predicting (S620), based at least on the input surview image(V0), one or more predicted 3D surview images (Vj, j>0) representable of the object in other one or more predicted motion states, and the object in the one or more predicted motion states occupying one or more other portions of space, and
providing (S630) at least the one or more predicted surview images (Vj) for the said facilitating of the said planned raw data acquisition.

12. Computing-implemented method of training, based on training data, a machine learning model for use in planning of a raw data acquisition by a tomographic imaging apparatus (IA), the model, when trained capable of predicting, given a surview image of a movable object at a first motion state, one or more such predicted surview images for the object at different one or more motion states.

13. Computing-implemented method of obtaining training data for use in a method of training, based on the training data to obtained, a machine learning model for use in planning of a raw data acquisition by a tomographic imaging apparatus (IA), the model, when trained capable of predicting, given a surview image of a movable object at a first motion state, one or more such predicted surview images for the object at different one or more motion states.

14. A computer program element, which, when being executed by at least one data processing system, is adapted to cause the data processing system (FS) to perform the method as per any one of claim 11, 12 or 13.

15. At least one computer readable medium (MEM) having stored thereon the program element of claim 14, or having stored thereon the model as per any one of claims 6-8.

**FIG. 1**

**FIG. 2**

FIG. 3

A)

M

LM

PAT

PS    LM

B)

M

**FIG. 4**

**FIG. 5**

**FIG. 6**

**FIG. 7**

## EUROPEAN SEARCH REPORT

Application Number

EP 23 18 4983

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 838 157 A1 (SIEMENS HEALTHCARE GMBH [DE]) 23 June 2021 (2021-06-23) | 1,2,6-15 | INV. A61B6/03 |
| Y | * abstract * | 3 | |
| A | * paragraph [0025] – paragraph [0038] * * paragraph [0071] – paragraph [0073] * * figure 1, * | 4,5 | |
| Y | US 2004/202283 A1 (OKUMURA MIWA [JP] ET AL) 14 October 2004 (2004-10-14) | 3 | |
| A | * abstract * * paragraph [0031] – paragraph [0039] * * figures 2-3 * | 1,2,4-15 | |
| A | US 2009/285357 A1 (KHAMENE ALI [US] ET AL) 19 November 2009 (2009-11-19) * abstract * * paragraph [0016] * * paragraph [0049] – paragraph [0052] * * paragraph [0058] – paragraph [0065] * | 1-15 | |

TECHNICAL FIELDS
SEARCHED     (IPC)

A61B

The present search report has been drawn up for all claims

| Place of search | Date of completion of the search | Examiner |
|---|---|---|
| Munich | 20 November 2023 | Montes, Pau |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

## ANNEX TO THE EUROPEAN SEARCH REPORT
## ON EUROPEAN PATENT APPLICATION NO.

EP 23 18 4983

This annex lists the patent family members relating to the patent documents cited in the above-mentioned European search report.
The members are as contained in the European Patent Office EDP file on
The European Patent Office is in no way liable for these particulars which are merely given for the purpose of information.

20-11-2023

| Patent document cited in search report | | Publication date | Patent family member(s) | | Publication date |
|---|---|---|---|---|---|
| EP 3838157 | A1 | 23-06-2021 | NONE | | |
| US 2004202283 | A1 | 14-10-2004 | CN | 1515228 A | 28-07-2004 |
| | | | JP | 2004180715 A | 02-07-2004 |
| | | | US | 2004202283 A1 | 14-10-2004 |
| US 2009285357 | A1 | 19-11-2009 | EP | 2285279 A2 | 23-02-2011 |
| | | | US | 2009285357 A1 | 19-11-2009 |
| | | | WO | 2009142680 A2 | 26-11-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL**. Machine Learning. McGraw-Hill, 1997, 2, 6

- **IAN GOODFELLOW et al.** Generative Adversarial Nets. *arXiv: 1406.2661v1*, 10 June 2014